(19) Europäisches Patentamt
European Patent Office
Office européen des brevets

(11) EP 3 616 682 A1

(12) **EUROPEAN PATENT APPLICATION**
published in accordance with Art. 153(4) EPC

(43) Date of publication:
**04.03.2020 Bulletin 2020/10**

(21) Application number: **18896703.8**

(22) Date of filing: **26.12.2018**

(51) Int Cl.:
*A61K 8/73* (2006.01) *A61Q 1/02* (2006.01)

(86) International application number:
**PCT/JP2018/047831**

(87) International publication number:
**WO 2019/131755 (04.07.2019 Gazette 2019/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.12.2017 JP 2017249854**

(71) Applicant: **Japan Corn Starch Co., Ltd.**
**Tokyo 107-0052 (JP)**

(72) Inventors:
- **MATSUYAMA Yuka**
  **Hekinan-shi, Aichi 4478608 (JP)**
- **SAKAKIBARA, Yuri**
  **Hekinanshi, Aichi (JP)**

(74) Representative: **Richly & Ritschel Patentanwälte PartG mbB**
**Sattlerweg 20**
**51429 Bergisch Gladbach (DE)**

(54) **COSMETIC COMPOSITION, COSMETIC PRODUCT, AND METHOD FOR PRODUCING COSMETIC COMPOSITION**

(57) This cosmetic composition (1) includes a starch particle (11) and a lubricant that covers at least a portion of a surface of the starch particle (11) and contains a fatty acid, in which a mass of the lubricant is 0.3 mass% to 20 mass% with respect to the total mass of the starch particle and the lubricant.

*FIG. 1*

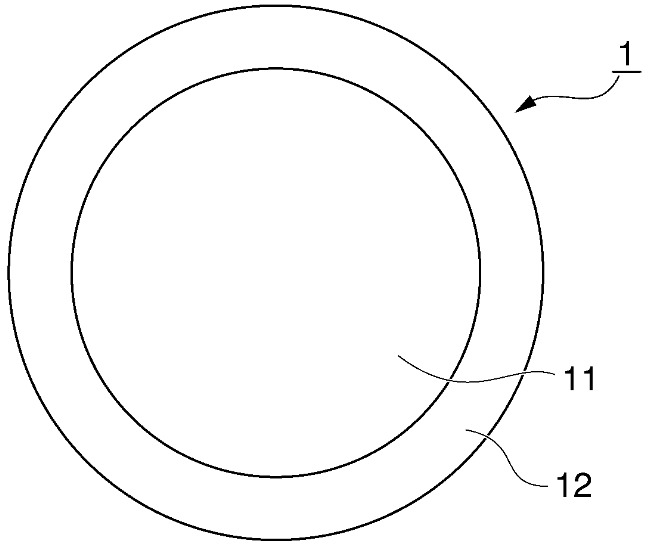


EP 3 616 682 A1

## Description

### TECHNICAL FIELD

The present invention relates to a cosmetic composition, a cosmetic product, and a method for producing a cosmetic composition.

Priority is claimed on Japanese Patent Application No. 2017-249854, filed on December 26, 2017, the content of which is incorporated herein by reference.

### BACKGROUND ART

In a cosmetic product such as foundations, lipsticks, and eye shadows, for the purpose of imparting a moisture retention sensation and softness to the skin and improving slipping during use, a feeling agent is added. Examples of the feeling agent include spherical particles made of a synthetic material such as silicon, nylon, and a polymethyl methacrylate resin.

In addition, spherical particles made of the synthetic material, which are generally called scrubs and are blended in a cleansing cosmetic product used for the purpose of peeling or washing the human body or a part of the human body, may be used.

If the spherical particles made of the synthetic material are discharged into the natural environment, the spherical particles remain in the natural world without being decomposed. Therefore, there are concerns about marine pollution and adverse effects on the human body. As a countermeasure against this, for example, in the United States, the use of solid plastic fine particles (hereinafter, referred to as plastic microbeads) with a size of less than 5 mm as the scrub is regulated.

Therefore, as an alternative to plastic microbeads, attention has been focused on products formed of biodegradable and naturally derived materials.

As an example of using naturally derived spherical particles or powder for a cosmetic product, Patent Document 1 discloses that microcrystalline cellulose is surface-treated with metal soap or hydrogenated lecithin, to be used as a cosmetic powder. Patent Document 2 discloses forming a cosmetic composition in which one or more kinds of particles selected from plants, animals, microorganisms, extracts thereof, and metabolites thereof are used as a base material.

### Prior Art Literature

#### Patent Documents

Patent Document 1: Japanese Unexamined Patent Application, First Publication No. 2003-146829
Patent Document 2: Japanese Unexamined Patent Application, First Publication No. 2017-52706

### SUMMARY OF THE INVENTION

#### Problems to be Solved by the Invention

The cosmetic powder disclosed in Patent Document 1 is produced by a so-called wet method in which production is performed by mixing in water. Therefore, it is required to remove water from the product or to dry the product, so there is room for improvement in terms of the number of steps and production cost. A naturally derived material cosmetic composition that is more nearly spherical and has improved sensation upon use is required.

The cosmetic composition disclosed in Patent Document 2 is produced by introducing a base material with a collapsed shape into a stirring granulator to improve the circularity factor and the shape factor, but a naturally derived material cosmetic composition of which sensation upon use is improved is required. In addition, with respect to a naturally derived material cosmetic composition, while biodegradability when being discarded is important, it is required to have anti-corrosion properties when being used as a cosmetic product.

The present invention has been made in view of the above circumstances, and an object thereof is to provide a naturally derived material cosmetic composition that can be produced efficiently, has good spreadability on the skin, and has excellent anti-corrosion properties, a cosmetic product including the cosmetic composition, and a method for producing a cosmetic composition.

Means for Solving the Problems

The present invention has the following aspects.

[1] A cosmetic composition, including: a starch particle; and a lubricant that covers at least a portion of a surface of the starch particle and containing a fatty acid, in which a mass of the lubricant with respect to the total mass of the starch particle and the lubricant is 0.3 mass% to 20 mass%.
[2] The cosmetic composition according to [1], in which the lubricant is at least one substance selected from the group consisting of metal soaps and waxes derived from vegetable oils and fats.
[3] The cosmetic composition according to [1] or [2], in which an average friction coefficient of the cosmetic composition is 0.30 to 0.55.
[4] The cosmetic composition according to any one of [1] to [3], in which an average particle diameter of the cosmetic composition is at least 2 μm but not more than 80 μm.
[5] The cosmetic composition according to any one of [1] to [4], in which a flowability index of the cosmetic composition based on the Carr's index table is at least 60.
[6] The cosmetic composition according to any one of claims [1] to [5], in which the lubricant covers at least 90% of the surface of the starch particle.
[7] The cosmetic composition according to any one of claims [1] to [6], in which the starch particle is corn starch, and the lubricant is zinc stearate.
[8] The cosmetic composition according to any one of [1] to [7], in which a specific surface area of the cosmetic composition is at least 0.60 $m^2/g$ but not more than 0.85 $m^2/g$.
[9] The cosmetic composition according to any one of [1] to [8], in which an average friction coefficient of the cosmetic composition is 0.30 to 0.55.
[10] The cosmetic composition according to any one of [1] to [9], in which an average particle diameter of the cosmetic composition is at least 10 μm but not more than 20 μm.
[11] The cosmetic composition according to any one of [1] to [10], in which the starch particle and the lubricant are composited.
[12] A cosmetic product including: the cosmetic composition according to any one of [1] to [11].
[13] A method for producing a cosmetic composition, including: refining particles of a lubricant containing a fatty acid and covering at least a portion of a surface of a starch particle with the lubricant by dry-mixing the starch particle and the lubricant.
[14] A method for producing a cosmetic composition according to [13], in which the starch particle and the lubricant are dry-mixed such that a mass of the lubricant is 0.3 mass% to 20 mass% with respect to the total mass of the starch particle and the lubricant.
[15] The method for producing a cosmetic composition according to [13] or [14], in which the starch particle and the lubricant are dry-mixed by using a dry compounding machine or a high shear mixer.
[16] The method for producing a cosmetic composition according to any one of [13] to [15], in which an average particle diameter of the lubricant before the dry-mixing is 1.0 μm or more.
[17] The method for producing a cosmetic composition according to any one of claims [13] to [16], in which the dry-mixing is performed at 30°C or more.
[18] The method for producing a cosmetic composition according to any one of [13] to [17], in which the starch particle is corn starch, and the lubricant is zinc stearate.
[19] The method for producing a cosmetic composition according to any one of [13] to [18], in which the dry-mixing is performed at 30°C to 90°C.

Advantageous Effects of Invention

According to the present invention, it is possible to provide a naturally derived material cosmetic composition that can be produced efficiently, has good spreadability on the skin, and has excellent anti-corrosion properties, a cosmetic product including the cosmetic composition, and a method for producing a cosmetic composition.

BRIEF DESCRIPTION OF DRAWINGS

FIG. 1 is a schematic cross-sectional view of a cosmetic composition according to an aspect of the present invention.
FIG. 2 is an SEM image of the cosmetic composition according to the aspect of the present invention.
FIG. 3 is an SEM image of the cosmetic composition according to the aspect of the present invention.

FIG. 4 is an SEM image of the cosmetic composition according to the aspect of the present invention.
FIG. 5 is a graph presenting a particle size distribution of a cosmetic composition and untreated corn starch according to the aspect of the present invention.
FIG. 6 is a diagram illustrating intensity distribution of reflected light when the cosmetic composition according to the aspect of the present invention is irradiated with light.

BEST MODE FOR CARRYING OUT THE INVENTION

Hereinafter, embodiments of the present invention are described below. The following embodiments are merely examples for explaining the present invention, but the present invention is not intended to be limited only to these aspects. The present invention can be implemented in various aspects without departing from the gist of the present invention.
In the present specification, an "average particle diameter" is defined to be a value measured by using a laser diffraction or scattering particle size distribution measuring device. Specifically, by using a laser diffraction particle size distribution measuring device (produced by Shimadzu Corporation, SALD2300), 0.5 g of the measurement object is added to 10 ml of an ethanol solution to obtain a dispersion in which the measurement object is dispersed. The particle size distribution of the obtained dispersion is measured to obtain a volume-based cumulative particle size distribution curve. In the obtained cumulative particle size distribution curve, the value of the particle diameter ($D_{50}$) at 50% cumulation viewed from the fine particle side is defined as the average particle diameter.
A particle diameter $D_{10}$ is defined as a value of a particle diameter at 10% cumulation viewed from the fine particle side in the cumulative particle size distribution curve obtained as described above. A particle diameter $D_{90}$ is defined as a value of a particle diameter at 90% cumulation viewed from the fine particle side in the cumulative particle size distribution curve obtained as described above.

(Cosmetic composition)

The cosmetic composition according to one aspect of the present invention includes a starch particle and a lubricant that covers at least a portion of a surface of the starch particle and containing a fatty acid, in which a mass of the lubricant with respect to the total mass of the starch particle and the lubricant is 0.3 mass% to 20 mass%.
The starch in the present specification is a polysaccharide having an $\alpha$1-4 linked D-glucan as the main chain. A main component of the starch particle is amylose, which is an $\alpha$1-4 linked D-glucan, and amylopectin having amylose as the main chain and an $\alpha$1-6 bonded side chain added as a side chain. The ratio of amylose and amylopectin varies depending on the raw material, but generally the content of amylopectin is 70 to 80 mass% with respect to the entire starch particle. In the case of high amylose species, the content of the amylose is 60 to 70 mass% with respect to the entire starch particle.
Examples of the starch particle includes corn starch, rice starch, potato starch, tapioca starch, sweet potato starch, red bean starch, pea starch, and mung bean starch based on their origins. Corn starch is preferable because of the rounded shape thereof.
As the starch particles, the above materials may be mixed. For example, when corn starch and rice starch are mixed and used as raw materials, the mass of corn starch with respect to the total mass of the entire starch particles is 30 to 95 mass% and preferably 60 to 90 mass%.
The starch in the present specification may be chemically modified starch. The term chemically modified starch means starch obtained by enzymatically or chemically processing starch particles obtained from natural raw materials for the purpose of improving the original physical properties of starch (for example, high viscosity, gelation properties in a case of being cooled). The enzymatic or chemical processing for starch may be one kind, or a plurality of enzymatic or chemical processing may be combined. The chemically modified starch that can be used in the present application includes starch acetate, oxidized starch, phosphorylated starch, phosphate cross-linked starch, hydroxypropyl starch, carboxymethyl starch, hydroxyethyl starch, esterified starch, graft polymerized starch, cationic starch, dextrin, hydrolyzed starch, hydrolyzed hydrogenated starch, and hydroxypropyl phosphorylated starch. Further, the chemically modified starch may be a chemically modified starch derivative obtained by adding a metal salt to chemically modified starch such as sodium acrylate graft starch, starch calcium octenyl succinate, starch sodium octenyl succinate, and starch aluminum octenyl succinate.
The starch in the present specification may be modified starch obtained by subjecting starch particles obtained from natural raw materials to a physical treatment. Examples of the modified starch that can be used in the present application include pregelatinized starch obtained by gelatinizing starch and wet heat-treated starch.
The average particle diameter of the starch particle is 2 $\mu$m to 80 $\mu$m, and preferably 5 $\mu$m to 20 $\mu$m. If the average particle diameter is 5 $\mu$m to 20 $\mu$m, the sensation on the skin is satisfactory.
In the present specification, the starch used as a raw material may be a commercially available product, or can be produced from corn, rice, potato, tapioca, and the like according to a well-known method corresponding thereto. In

addition, the chemically modified starch used as a raw material can be easily produced or obtained by using a commercially available product or by subjecting the starch to an appropriate chemical treatment according to a well-known method corresponding thereto.

The lubricant contains at least a fatty acid. In the present specification, a case where the lubricant contains a fatty acid is not limited to a case where the lubricant is a fatty acid salt or a fatty acid ester or a case where the lubricant includes a fatty acid or a fatty acid ester as a component, and also includes a case where the lubricant includes a component including a fatty acid salt or a fatty acid ester in a part of the structure.

The lubricant includes at least one substance selected from the group consisting of metal soaps and waxes derived from vegetable oils and fats. Here, the "metal soap" is a general term for long-chain fatty acids (for example, fatty acids having 12 to 18 carbon atoms) and salts of metals other than sodium and potassium.

For example, the lubricant contains at least one metal soap represented by Formula (I).

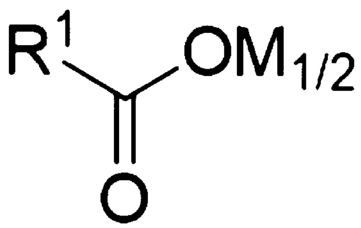

(I)

(in the formula, $R^1$ is selected from the group consisting of a saturated hydrocarbon group having 11 to 17 carbon atoms which may have a branch and an unsaturated hydrocarbon group having 11 to 17 carbon atoms which may have a branch (the group represented by $R^1$ each independently may be substituted with at least one group selected from the group consisting of a hydroxy group, an alkoxyl group, a carboxyl group, and an oxo group), and M is a divalent magnesium ion, zinc ion, or calcium ion.)

Examples of the saturated hydrocarbon group having 11 to 17 carbon atoms include an n-undecyl group, an n-dodecyl group, an n-tridecyl group, an n-tetradecyl group, an n-hexadecyl group, and an n-heptadecyl group.

Examples of the unsaturated fatty acid group having 11 to 17 carbon atoms include an undecenyl group, a dodecenyl group, a tridecenyl group, a tetradecenyl group, a hexadecenyl group, and a heptadecenyl group.

Examples of the metal soap represented by Formula (I) include magnesium stearate, zinc stearate, calcium stearate, aluminum stearate, sodium stearate, zinc laurate, potassium laurate, sodium myristate, zinc myristate, zinc palmitate, and a mixture thereof. As the metal soap represented by Formula (I), magnesium stearate, zinc stearate, and calcium stearate are preferable, and zinc stearate is more preferable.

The wax derived from vegetable oils and fats is also referred to as a wax, and is a substance including ester of a higher fatty acid derived from a plant (for example, a fatty acid having 12 to 24 carbon atoms) and a monovalent or divalent higher alcohol (for example, an alcohol having 8 to 22 carbon atoms) as the main component and is a solid substance at room temperature (for example, 23°C). Here, the "main component" is 60 mass% or more, preferably 80 mass% or more, and more preferably 90 mass% or more, and may be 100 mass% with respect to the total mass of the wax. More specifically, carnauba wax, and candelilla wax and the like can be exemplified. Here, "carnauba wax" means wax extracted from the leaves and petioles of carnauba palm, and is a substance including an ester such as myricyl cerotate, as the main component. The "main component" has the aforementioned meaning. The "candelilla wax" means wax extracted from the stems of candelilla plants, and is a substance including hentriacontane (C31H64), as the main component.

In the present specification, the lubricant can be easily produced and obtained by using a commercially available product or according to a well-known method corresponding thereto.

In the present specification, the expression "a lubricant that covers at least a portion of the surface of the starch particle" means that the lubricant adheres to at least a portion of the surface of the starch particle, and the lubricant covers at least a portion of the starch particle. Here, the term "adhesion" includes a bonding state in which bonding is performed to a relatively easily removable degree according to an intermolecular force between the lubricant and the starch particle and includes a bonding state in which the lubricant and starch particle are pressed to each other, and the lubricant is immobilized on the surface of the starch particle.

The latter bonding state may also be referred to as "compositing a lubricant and a starch particle" in the present specification, and forming a lubricant layer on at least a portion of the surface of the starch particle by the "composite" is referred to as "coating" in some cases.

In the present specification, if the lubricant adheres to the surface of the starch particle to a relatively easily removable degree and covers at least a portion of the surface of the starch particle, the characteristics of starch particle can be modified to a state suitable for the present invention in some cases. However, in view of blending the cosmetic

composition according to the present invention in a cosmetic product to be used, it is more preferable that the lubricant and the starch particle be composited such that the structure of the cosmetic composition is maintained even inside the cosmetic product.

In one aspect of the present invention, the lubricant is deposited (also referred to as coating) as a lubricant layer on the surface of the starch particle by covering the surface of the starch particle so as to be composited with the lubricant.

The mass of the lubricant included in the cosmetic composition is 0.3 mass% to 20 mass%, preferably 0.5 mass% to 15 mass%, more preferably 1 mass% to 10 mass%, and even more preferably 1 mass% to 7 mass% with respect to the total mass of the starch particle and the lubricant. If the mass of the lubricant is at least 0.3 mass% with respect to the total mass of the starch particle and the lubricant, a sufficient amount of the lubricant adheres to the surface of the starch particle, spreadability on the skin when the cosmetic component is added to the cosmetic product can be improved. If the mass of the lubricant is at least 5 mass%, the anti-corrosion properties of the cosmetic composition can be further improved. If the mass of the lubricant is not more than 20 mass% with respect to the total mass of the starch particle and the lubricant, the flowability is hardly inhibited by the lubricant that does not adhere to the surface of the starch particle.

Because the surface of the starch particle is covered with the lubricant layer, the cosmetic composition has higher flowability as a powder than a case where the starch particle is a single substance. Specifically, the flowability index based on the Carr's index table is at least 60, preferably at least 63, and even more preferably at least 65. The higher the flowability index, the better, but the upper limit is, for example, 85.

The flowability index based on the Carr's index table is obtained by indexing the measurement results of degree of compression, angle of repose, angle of spatula, and degree of uniformity of the measurement object based on the Carr's index table and calculating the total of the indices (see Carr, R. L.: Evaluating flow properties of solids. Chem. Eng. 1965; 72: 163-168). The flowability is evaluated as "low" in a case where the total index is at least 40 but less than 60, "normal" in a case where the total index is at least 60 but less than 70, "high" in a case where the total index is at least 70 but less than 80, and "pretty high" in a case where the total index is at least 80 but less than 90. The method for measuring the degree of compression, angle of repose, angle of spatula, and degree of uniformity is as follows.

[Degree of compression]

The mass per unit volume in a state where a cosmetic composition which is a measurement sample is dropped to fill a 100 $cm^3$ measurement container while passing through a sieve, and the container is filled with the measurement sample by dropping is referred to as "loose bulk density", and the mass per unit volume of the sample after tapping is repeated 200 times with a stroke length of 50 mm is referred to as "hard bulk density". The compression density is calculated by Expression (1).

Degree of compression = [(hard bulk density - loose bulk density) / hard bulk density] $\times$ 100(%) ... (1)

[Angle of repose]

100 g of the cosmetic composition is dropped through a funnel, and the deposit is formed on a base plate located 7 cm below the tip of the funnel. Then, an angle formed by an inclined surface with a horizontal plane is measured.

[Angle of spatula]

A spatula (length: 8 cm, width: 2.2 cm) is inserted into the bottom of the powder deposit and the spatula is gently lifted out of the deposit. Next, the spatula is tapped and an angle formed by the inclined surface with the horizontal plane when the deposit is formed is measured.

[Degree of uniformity]

A particle diameter ($D_{10}$) at 10% cumulation of the cosmetic composition when being viewed from the fine particle side, which is measured by using a laser diffraction or scattering particle size distribution measuring device, is defined as 10% cumulative diameter. A particle diameter ($D_{60}$) at 60% cumulation of the cosmetic composition when being viewed from the fine particle side, which is measured by using a laser diffraction or scattering particle size distribution measuring device, is defined as 60% cumulative diameter. Degree of uniformity was calculated from a ratio ($D_{10}/D_{60}$) of 10% cumulative diameter to 60% cumulative diameter.

The average particle diameter $D_{50}$ of the cosmetic composition according to one aspect of the present invention is at least 2 $\mu$m but not more than 80 $\mu$m, preferably at least 5 $\mu$m but not more than 40 $\mu$m, more preferably at least 5 $\mu$m but not more than 20 $\mu$m, and even more preferably at least 10 $\mu$m but not more than 18 $\mu$m. If the average particle

diameter of the cosmetic composition is at least 2 μm but not more than 80 μm the sensation on the skin when used as a cosmetic becomes good. The average particle diameter $D_{50}$ is a particle diameter at 50% cumulation of the cosmetic composition viewed from the fine particle side, which is measured by using a laser diffraction or scattering particle size distribution measuring device.

($D_{90}$-$D_{10}$) which is a difference between the particle diameter $D_{10}$ and the particle diameter $D_{90}$ of the cosmetic composition according to one aspect of the present invention is preferably not more than 20 μm, more preferably not more than 16 μm, and even more preferably not more than 10 μm. As the difference between the particle diameter $D_{10}$ and the particle diameter $D_{90}$ is smaller, the variation of the particle diameter is smaller. If the difference between the particle diameter $D_{10}$ and the particle diameter $D_{90}$ is not more than 10 μm, the sensation on the skin when the cosmetic composition is used as a cosmetic product is improved. That is, if the difference between the particle diameter $D_{10}$ and the particle diameter $D_{90}$ is not more than 10 μm, the cosmetic composition is excellent for the use as a feel modifier. As the reason why the difference between the particle diameter $D_{10}$ and the particle diameter $D_{90}$ of the cosmetic composition according to one aspect of the present invention is not more than 10 μm, it is considered that, in a process of covering at least a portion of the surface of the starch particle in the production of the cosmetic composition with the lubricant, the secondary particles which are aggregates of the primary particles of the starch particles are pulverized.

The lower limit of the difference between the particle diameter $D_{10}$ and the particle diameter $D_{90}$ of the cosmetic composition is not particularly limited, but considering the particle size distribution of the primary particles of starch particles is, for example, 3 μm.

The upper and lower limits of the difference between the particle diameter $D_{10}$ and the particle diameter $D_{90}$ of the cosmetic composition may be arbitrarily combined. For example, the difference between the particle diameter $D_{10}$ and the particle diameter $D_{90}$ of the cosmetic composition is preferably at least 3 μm but not more than 20 μm and more preferably at least 3 μm but not more than 16 μm.

As an example, the difference between the particle diameter $D_{10}$ and the particle diameter $D_{90}$ of the cosmetic composition in a case where the starch particles are corn starch and the lubricant includes zinc stearate is preferably at least 7 μm but not more than 10 μm and more preferably at least 7 μm but not more than 9 μm. At this time, the frequency of about at least 1 μm but not more than 8 μm derived from the particles of the lubricant is preferably not more than 0.1 and more preferably not more than 0.05. This is because, if almost all of the lubricant covers the starch particle and is composited, the frequency of particle diameter derived from the lubricant should be extremely small.

The average friction coefficient of the cosmetic composition according to one aspect of the present invention is at least 0.30 but not more than 0.55 and preferably at least 0.30 but not more than 0.50. If the average friction coefficient of the cosmetic composition is at least 0.30 but not more than 0.55, when the cosmetic composition is used as a cosmetic, the spreadability of the cosmetic on the skin is improved.

The average friction coefficient of the cosmetic composition can be measured with a powder layer shear force measuring device. Specifically, the shear stress (friction force) generated when two upper and lower cells of a powder layer shear force measuring device (produced by Nano Seeds Corporation, NS-S500 type) is filled with the cosmetic composition and shifted while a load of 90 kPa is applied is measured.

The average dynamic friction coefficient of the cosmetic composition according to one aspect of the present invention is at least 0.30 but not more than 0.48, preferably at least 0.30 but not more than 0.45, and more preferably at least 0.30 but not more than 0.42. If the average dynamic friction coefficient of the cosmetic composition is 0.30 to 0.48, when the cosmetic composition is used as a cosmetic, the spreadability of the cosmetic on the skin is improved.

The average dynamic friction coefficient of the cosmetic composition can be measured by a static or dynamic friction measuring machine. Specifically, 30 mg of the cosmetic composition is weighed and placed on a 5 cm × 2 cm double-sided tape fixed on a slide glass and uniformly applied by a fingertip with a nitrile glove to prepare a sample. The cosmetic composition is slid by using a static or dynamic friction measuring machine (produced by Trinity-Lab Inc., TL201Ts), at room temperature of 22°C to 25°C and relative humidity of 45% to 55%, by employing a urethane tactile contactor (contact area: 1.5 $cm^2$), under the conditions of a sliding speed of 10 mm/sec, a sliding distance of 20 mm, and a vertical load of 30 g, and the surface friction is measured.

For the average dynamic friction coefficient, the average value of the dynamic friction coefficients in a sliding distance range of 5 to 12 mm is obtained from the measurement results, measurement is performed five times, and the average dynamic friction coefficient is calculated to obtain the average value.

In the present specification, compared with the state where the lubricant is adhered to the surface of the starch particle so as to be relatively easily removable, in a state where the lubricant and the starch particle are composited to form a lubricant layer, the surface of the lubricant layer tends to be smooth. Further, since the lubricant and the starch particle are composited to form the lubricant layer, the particle tends to be closer to a spherical shape than the starch particle as a raw material.

The specific surface area of the cosmetic composition according to one aspect of the present invention varies according to the kind of the starch particle, but is preferably at least 0.55 $m^2/g$ but not more than 10.00 $m^2/g$ and more preferably at least 0.60 $m^2/g$ but not more than 2.00 $m^2/g$. If the specific surface area of the cosmetic composition is at

least 0.60 $m^2/g$ but not more than 2.00 $m^2/g$, the surface of the cosmetic composition is smooth, and the sensation on the skin when the cosmetic composition is added to a cosmetic product is improved.

For example, the specific surface area of the cosmetic composition when the starch particle is a corn starch is preferably at least 0.58 $m^2/g$ but not more than 0.95 $m^2/g$ and more preferably at least 0.60 $m^2/g$ but not more than 0.85 $m^2/g$.

In the present specification, the "specific surface area" is a value measured by the Brunauer-Emmet-Teller (BET) method. In the measurement of the BET specific surface area, nitrogen gas is used as the adsorption gas. The BET specific surface area is the value obtained by drying about 1 g of the powder of the measurement sample in a nitrogen atmosphere at 50°C for 240 minutes and subsequently measuring using the BET specific surface area meter (produced by Mountech Co.Ltd., Macsorb (registered trademark)).

The cosmetic composition according to one aspect of the present invention preferably has irregular reflectivity. The irregular reflectivity can be confirmed by measuring the reflection intensity distribution with a three-dimensional goniophotometer. Specifically, the irregular reflectivity can be confirmed by measuring the intensity distribution of the reflected light in the range of -90° to 90° by using a three-dimensional goniophotometer (produced by Murakami Color Research Laboratory Co., Ltd., GP-200) when the surface uniformly applied with about 0.5 g of the powder to be measured is irradiated with light having an incident angle of 45°. In the cosmetic composition according to one aspect of the present invention, when the maximum value of reflected light measured in the range of -90° to 90° is set to 85%, the intensity of the reflected light is preferably at least 30% and more preferably at least 40% with respect to the total range of -70° to 70°. Here, in the present specification, the "intensity of reflected light with respect to the total range of -70° to 70°" does not include intensity of reflected light at the incident angle of irradiation light due to the nature of the measurement method.

For example, in the cosmetic composition when the starch particle is corn starch and the lubricant includes zinc stearate, if the maximum value of reflected light measured in the range of -90° to 90° is set to 85%, the intensity of the reflected light is preferably at least 50% and more preferably at least 60% with respect to the total range of -70° to 70°.

The cosmetic composition according to one aspect of the present invention is described by using FIG. 1. FIG. 1 is a schematic cross-sectional view of a cosmetic composition according to one aspect of the present invention. A cosmetic composition 1 includes a starch particle 11 and a lubricant layer 12 that covers a surface of the starch particle. FIG. 1 shows an example where the lubricant layer 12 covers the entire surface of the starch particle 11.

In the cosmetic composition according to one aspect of the present invention, the starch particle is covered with the lubricant, so anti-corrosion properties are exhibited compared with a case of only the starch particle. It is considered that since the fatty acid included in the lubricant has water repellency and hydrophobicity, the permeation of moisture into the starch particle is suppressed, and the growth of microorganisms is suppressed. Thus, the cosmetic composition according to one aspect of the present invention has anti-corrosion properties even when a natural material such as a starch particle is used.

The cosmetic composition according to one aspect of the present invention can be used as a cosmetic composition in a cosmetic product. In the cosmetic composition according to one aspect of the present invention, the average friction coefficient is small as described above, so the cosmetic composition can be used as a feeling agent that improves slipperiness particularly in a powder cosmetic product.

The cosmetic composition according to one aspect of the present invention can also be used as a scrub for the purpose of peeling or washing a human body or a part of the human body.

The cosmetic composition according to one aspect of the present invention may only include a starch particle and a lubricant layer that covers the surface of the starch particle.

(Method for producing cosmetic composition)

Hereinafter, a method for producing a cosmetic composition according to one aspect of the present invention is described.

The method for producing a cosmetic composition according to one aspect of the present invention includes refining particles of a lubricant containing a fatty acid and covering at least a portion of a surface of a starch particle with the lubricant by dry-mixing the starch particle and the lubricant.

In the dry-mixing of the starch particle and the lubricant, a high shear mixer, a dry compounding machine, or the like is used. When the starch particle and the lubricant are mixed while applying high shear force by using a high shear mixer, a dry compounding machine, or the like, a cosmetic composition in which the starch particle and the lubricant are composited can be produced.

The high shear mixer is a device that applies shear force to the powder between the paddle, blades, or the like, which is attached to the tip of the shaft extending perpendicularly from the central axis inside the device, and the wall of the device to adhere child particles (lubricant) to the surfaces of mother particles (that is, the starch particle surface) while mixing. Specific examples include a high shear mixer (produced by Nippon Coke Kogyo Co., Ltd., FM

mixer). If the starch particle and the lubricant are dry-mixed by using the high shear mixer, the starch particle and the lubricant are mixed such that the mass of the lubricant is 0.3 mass% to 20 mass% with respect to the total mass of the starch particle and the lubricant. If the mass of the lubricant is at least 0.3 mass% with respect to the total mass of the starch particle and the lubricant, a sufficient amount of the lubricant can be adhered to the starch particle surface. If the mass of the lubricant is not more than 20 mass% with respect to the total mass of the starch particle and the lubricant, the inhibition of the flowability caused by the lubricant that is not adhered to the starch particle surface hardly occurs.

If dry-mixing is performed by the high shear mixer, compared with a case where the dry-mixing is performed by the dry compounding machine, the temperature inside the device during the mixture is generally lower. For example, the temperature during the dry-mixing by the high shear mixer is 30°C to 60°C. If dry-mixing is performed at a higher temperature, a hot water jacket (for example, a water temperature of 75°C) can be fitted to the high shear mixer. In this case, the temperature during the dry-mixing is preferably 60°C to 90°C. As in the case of using a dry compounding machine, the temperature during the dry-mixing is set according to the melting point of the lubricant, and it is preferable to be set from a temperature at which the lubricant softens to a temperature lower than the melting point of the lubricant. By setting the temperature during mixing in this manner, the lubricant is smoothly ground and almost uniformly covers the surface of the starch particle to composite the starch particle and the lubricant.

If the starch particle and the lubricant are dry-mixed by using a high shear mixer, the treatment is performed preferably at a rotational speed of 3,000 to 7,000 rpm for 1 to 60 minutes and more preferably at a rotational speed of 4,000 to 7,000 rpm for 5 to 30 minutes.

The dry compounding machine is a device that can apply a stronger shearing force than the high shear mixer and can treat harder materials than the high shear mixer. Specifically, examples of the composite treating machine (produced by Nippon Coke & Engineering Co., Ltd., COMPOSI). If the starch particle and the lubricant are dry-mixed by the dry compounding machine, the starch particle and the lubricant are mixed such that the mass of the lubricant is 0.3 mass% to 20 mass% with respect to the total mass of the starch particle and the lubricant. If the mass of the lubricant is at least 1.0 mass% with respect to the total mass of the starch particle and the lubricant, a sufficient amount of the lubricant can be adhered to the starch particle surface. If the mass of the lubricant is not more than 20 mass% with respect to the total mass of the starch particle and the lubricant, the inhibition of the flowability caused by the lubricant that is not adhered to the starch particle surface hardly occurs.

In the case where dry-mixing is performed by the dry compounding machine, the temperature in the tank during dry-mixing depends on the scale of the device but is 30°C to 100°C, preferably 60°C to 98°C, and even more preferably 85°C to 95°C. The temperature during dry-mixing is set according to the melting point of the lubricant, and is preferably set from a temperature at which the lubricant softens to a temperature lower than the melting point of the lubricant. By setting the temperature during mixing in this manner, the lubricant is smoothly ground and almost uniformly covers the surface of the starch particle to composite the starch particle and the lubricant.

In the case where dry-mixing is performed by the dry compounding machine, if there is a concern in that the temperature in the tank becomes higher than the above temperature range, the temperature in the tank can be adjusted, for example, by fitting a cooling water jacket (for example, a water temperature of 4°C) to the dry compounding machine.

In the case where the starch particle and the lubricant are dry-mixed by using a dry compounding machine, the treatment is performed preferably for 1 to 60 minutes at a rotational speed of 2,000 to 6,000 rpm and more preferably at 5 to 30 minutes at a rotational speed of 2,500 to 5,000 rpm.

By mixing the starch particle and the lubricant while a high shearing force is applied, the starch particle and the lubricant collide such that the starch particle refine the lubricant. Since the lubricant contains a fatty acid, the lubricant adheres to the surface of the starch particle due to the oil absorption of the starch particle. Since the lubricant is sufficiently refined with respect to the starch particle diameter, the surface of the starch particle can be covered almost uniformly. As described above, the lubricant does not necessarily have to cover the entire starch particle surface and may cover at least 90% of the entire starch particle surface.

The average particle diameter of the lubricant before dry-mixing may be at least 1.0 μm. In the method for producing a cosmetic composition according to one aspect of the present invention, since the starch particle and the lubricant are mixed while a high shearing force is applied to refine the lubricant, a refining treatment need not be performed on the lubricant before the mixing. Accordingly, the average particle diameter of the lubricant before dry-mixing may be at least 20 μm. Of course, the dry-mixing may be performed by using the lubricant refined to be not more than 20 μm.

In the method for producing a cosmetic composition according to one aspect of the present invention, since the starch particle and the lubricant are dry-mixed, the production amount per batch is more than that of wet-mixing. Further, a solvent recovery step or a drying step essential in wet mixing is unnecessary.

(Cosmetic product)

The cosmetic product according to one aspect of the present invention includes the cosmetic composition. The cosmetic product according to one aspect of the present invention is described below.

Examples of the form of the cosmetic product according to one aspect of the present invention include various dosage forms such as a solid agent or a liquid agent.

Examples of the cosmetic product of the solid preparation include makeup cosmetic products such as foundations, face powders, blushers, lipsticks, eye shadows, eyebrow pencils, and concealers. Examples of the form of the cosmetic product of the solid preparation according to the present invention include a cake shape, a stick shape, and a sphere shape.

Examples of the cosmetic product of the liquid preparation include skin lotions, emulsions, creams, essences, makeup bases, mascaras, sunscreen creams, hair cosmetic products, cleansing cosmetic products, shampoos, hair conditioners, hair treatment lotions, hair styling agents, hair tonics, and hair growth agents.

The cosmetic product according to one aspect of the present invention preferably includes 0.5 mass% to 60 mass% of the cosmetic composition according to one aspect of the present invention, more preferably includes 3 mass% to 30 mass%, and even more preferably includes 5 mass% to 10 mass% with respect to the total mass of the cosmetic product.

The average dynamic friction coefficient of the cosmetic product of the solid preparation containing the cosmetic composition according to one aspect of the present invention is preferably not more than 0.60 and more preferably not more than 0.59. The preferable range of the average dynamic friction coefficient varies depending on the type of cosmetic product of the solid preparation. For example, if the cosmetic product of the solid preparation is a blusher, the range of the average dynamic friction coefficient is preferably not more than 0.54, more preferably not more than 0.53, and more preferably not more than 0.52. If the cosmetic product of the solid preparation is eye shadow, the range of the average dynamic friction coefficient is preferably not more than 0.60 and more preferably not more than 0.59. The lower limit value of the average dynamic friction coefficient of the cosmetic product of the solid preparation is not particularly limited but may be 0.2, for example.

If the cosmetic product of the solid preparation is eye shadow, the average dynamic friction coefficient can be measured by the method for measuring the average dynamic friction coefficient of the cosmetic composition above. If the cosmetic product of the solid preparation is a blusher, the same method for measuring the average dynamic friction coefficient of the cosmetic composition is used, except that one obtained by filling a metal dish with blusher and hardening the blusher is used as a sample, to cause the sliding distance to be 10 mm.

In addition to the above component, the cosmetic product according to one aspect of the present invention may contain an oil agent for the purpose of adjusting sensation, improving adhesion to the skin, and improving makeup durability. As the oil agent, any one may be used, provided that is generally used in a cosmetic product, and examples thereof include liquid paraffin, Vaseline, petrolatum, pristane, paraffin wax, microcrystalline wax, ozokerite, ceresin, carnauba wax, beeswax, lanolin, lanolin alcohol, liquid lanolin, full hard lanolin, polybutene, oleyl alcohol, isostearyl alcohol, octyldodecanol, cetanol, steryl alcohol, dimethylpolysiloxane, methylphenylpolysiloxane, oleic acid, isostearic acid, palmitic acid, stearic acid, isopropyl myristate, hexyl laurate, isopropyl palmitate, decyl oleate, octyldodecyl myristate, glycerol trioctanoate, glycerol triisostearate, glycerol triisopalmitate, olive oil, safflower oil, avocado oil, macadamia nut oil, jojoba oil, wheat germ oil, tea seed oil, egg yolk oil, and mink oil. One or more kinds thereof may be used.

In addition to the above components, the cosmetic product according to one aspect of the present invention contains powders other than the cosmetic composition for the purpose of a colorant, an ultraviolet-shielding agent, an excipient, and a sensation modifier. Such powder may be any powder that is generally used in a cosmetic product, and examples thereof include yellow iron oxide, red iron oxide, black iron oxide, titanium oxide, zinc oxide, titanium mica, chromium oxide, chromium hydroxide, bismuth oxychloride, ultramarine, bitumen, titanium oxide covered mica, cochineal covered mica, calamine covered mica, chromium oxide covered mica, calamine, a tar-based coloring agent, particulated zinc oxide, particulated titanium oxide, iron oxide, cerium oxide, zirconium oxide, silk powder, silica, talc, mica, and kaolin. One or more kinds thereof may be used. Powders thereof may be subjected to a surface treatment by a generally known treating agent to be used.

The cosmetic product according to one aspect of the present invention may include a surfactant. The surfactant is used for purposes such as a dispersing agent and a sensation modifier, and examples thereof include nonionic surfactants such as glycerin fatty acid esters and an alkylene glycol adduct thereof, anionic surfactants such as alkylbenzene sulfate, cationic surfactants such as an alkylamine salt, and an amphoteric surfactant such as lecithin. One or more kinds thereof may be used.

The cosmetic product according to one aspect of the present invention may include an ultraviolet absorbing agent. As the ultraviolet absorbing agent, those generally used in a cosmetic product may be used, and examples thereof include benzophenones such as 2-hydroxy-4-methoxybenzophenone, 2,4,6-trianilino-para-(carbo-2'-ethylhexyl-1'-oxy)-1,3,5-triazine, salicylic acids such as salicylic acid-2-ethylhexyl, PABAs such as paradihydroxypropyl ethylbenzoate, cinnamic acids such as paramethoxycinnamic acid-2-ethylhexyl, and dibenzoylmethanes such as 4-tert-4'-methoxydibenzoylmethane. One or more kinds thereof may be used.

A water-soluble polymer used in the present invention is contained for the purpose of improving adhesion to the skin, improving makeup durability, and improving sensation. Specific examples thereof include cellulose derivatives

such as methylcellulose and hydroxymethylcellulose, natural polymers such as sodium alginate, carrageenan, agar, and gelatin pectin, and a synthetic polymer such as polyvinyl alcohol, a carboxyvinyl polymer, an alkyl-added carboxyvinyl polymer, sodium polyacrylate, sodium polymethacrylate, polyacrylic acid glycerin ester, and polyvinylpyrrolidone. One or more kinds thereof may be used.

In addition to the above components, the cosmetic product according to one aspect of the present invention may be obtained by appropriately adding components that are generally used in a cosmetic product, for example, a surfactant, an oil-gelling agent, an ultraviolet-absorbing agent, a water-soluble polymer, an oil-soluble film forming agent, an aqueous component, a paraoxybenzoic acid derivative, a preservative such as phenoxyethanol, vitamins, cosmetic ingredients, fragrances, in a range that does not deteriorate the effects of the present invention.

A method for producing the cosmetic product according to one aspect of the present invention is not particularly limited, but examples thereof include a method for mixing the cosmetic composition and other powders, adding an oil agent or the like, if necessary, performing pulverization, and performing compression molding into a container such as a metal dish or a resin dish.

The cosmetic composition according to one aspect of the present invention may be as follows.

[1] A cosmetic composition including a corn starch and a lubricant that covers at least a portion of the surface of the corn starch and that is zinc stearate, in which a mass of the lubricant is 1 mass% to 10 mass% with respect to the total mass of the corn starch and the lubricant.

[2] The cosmetic composition according to [1], in which the corn starch and the lubricant are composited.

[3] The cosmetic composition according to [1] or [2], in which a specific surface area of the cosmetic composition is 0.60 $m^2/g$ to 0.85 $m^2/g$.

[4] The cosmetic composition according to any one of [1] to [3], in which an average friction coefficient of the cosmetic composition is 0.30 to 0.55.

[5] The cosmetic composition according to any one of [1] to [4], in which an average particle diameter of the cosmetic composition is at least 10 $\mu$m but not more than 20 $\mu$m.

[6] The cosmetic composition according to any one of [1] to [5], in which a flowability index of the cosmetic composition is at least 60 based on the Carr's index table.

[7] The cosmetic composition according to any one of [1] to [6], in which the lubricant covers at least 90% of the surface of the corn starch.

[8] A cosmetic product including the cosmetic composition according to any one of [1] to [7].

[9] A method for producing a cosmetic composition including refining particles of a lubricant containing zinc stearate and covering at least a portion of a surface of a corn starch with the lubricant by dry-mixing the corn starch and the lubricant.

[10] The method for producing a cosmetic composition according to [9], in which the corn starch and the lubricant are dry-mixed such that a mass of the lubricant is 0.3 mass% to 20 mass% with respect to a total mass of the corn starch and the lubricant.

[11] The method for producing a cosmetic composition according to [9] or [10], in which the corn starch and the lubricant are dry-mixed by using a dry compounding machine or a high shear mixer.

[12] The method for producing a cosmetic composition according to any one of [9] to [11], in which an average particle diameter of the lubricant before the dry-mixing is at least 1.0 $\mu$m.

[13] The method for producing a cosmetic composition according to any one of [9] to [12], in which the dry-mixing is performed at 30°C to 90°C.

## EXAMPLES

Hereinafter, examples are provided to specifically describe the present invention, but the present invention is not limited by the following description.

(Evaluation of flowability of cosmetic composition based on the Carr's index table)

A flowability index based on the Carr's index table was obtained by indexing measurement results of degree of compression, angle of repose, angle of spatula, and degree of homogeneity of a measurement object based on the Carr's index table and calculating the total index thereof. The flowability is evaluated to be "low" in a case where the total index is 40 to 59, "normal" in a case where the total index is 60 to 69, and "high" in a case where the total index is 70 to 79. The methods for measuring the degree of compression degree, the angle of repose, the angle of spatula, and the degree of uniformity were as below.

[Degree of Compression]

A state where a cosmetic composition which was a measurement sample was dropped to fill a 100 $cm^3$ measurement container while passing through a sieve, and the container was filled with the measurement sample by dropping is referred to as "loose bulk density", and a sample volume after the container was capped and tapping is repeated 200 times with a stroke length of 50 mm is referred to as "hard bulk density". The compression density was calculated by Formula (1).

$$\text{Degree of compression} = (\text{hard bulk density} - \text{loose bulk density}) / \text{hard bulk density} \times 100(\%) \quad ...\ (1)$$

[Angle of repose]

100 g of the cosmetic composition was dropped through the funnel, and the deposit was formed on the base plate located 7 cm below the tip of the funnel. Then, an angle formed by the inclined surface with the horizontal plane was measured.

[Angle of Spatula]

A spatula (length: 8 cm, width: 2.2 cm) was inserted into the bottom of the powder deposit and the spatula was gently lifted out of the deposit. Next, the spatula was slightly tapped, and an angle formed by the inclined surface with the horizontal plane when the deposit was formed was measured.

[Degree of uniformity]

A particle diameter ($D_{10}$) at 10% cumulation of the cosmetic composition when being viewed from the fine particle side, which is measured by using a laser diffraction or scattering particle size distribution measuring device, is defined as 10% cumulative diameter. A particle diameter ($D_{60}$) at 60% cumulation of the cosmetic composition when being viewed from the fine particle side, which is measured by using a laser diffraction or scattering particle size distribution measuring device, is defined as 60% cumulative diameter. Degree of uniformity was calculated from a ratio of 10% cumulative diameter to 60% cumulative diameter.

(Measurement of particle size distribution of cosmetic composition)

By using a laser diffraction particle size distribution measuring device (produced by Shimadzu Corporation, SALD2300), 0.5 g of the measurement object was added to 10 ml of an ethanol solution to obtain a dispersion liquid in which the measurement object was dispersed. The particle size distribution was measured of the obtained dispersion liquid, and the volume-based cumulative particle size distribution curve was obtained.

(Measurement of average friction coefficient of cosmetic composition)

As the average friction coefficient of the cosmetic composition, the shear stress (frictional force) generated when two upper and lower cells of a powder layer shear force measuring device (produced by Nano Seeds Corporation, NS-S500 type) was filled with the cosmetic composition and shifted while a load of 90 kPa was applied was measured.

(Measurement of average dynamic friction coefficient of cosmetic composition or cosmetic product)

The average dynamic friction coefficient of the cosmetic composition or the cosmetic product was measured by a static or dynamic friction measuring machine (produced by Trinity-Lab Inc., TL201Ts).

[Test method 1]

30 mg of the measurement sample was weighed and placed on a 5 cm $\times$ 2 cm double-sided tape fixed on a slide glass and uniformly applied by a fingertip with a nitrile glove to prepare a sample. The cosmetic composition was slid by using a static or dynamic friction measuring machine (produced by Trinity-Lab Inc., TL201Ts), at room temperature of 22°C to 25°C and relative humidity of 45% to 55%, by employing a urethane tactile contactor (contact area: 1.5 $cm^2$),

under the conditions of a sliding speed of 10 mm/sec, a sliding distance of 20 mm, and a vertical load of 30 g, and the surface friction was measured. For the average dynamic friction coefficient, the average value of the dynamic friction coefficients in a sliding distance range of 5 to 12 mm was obtained from the measurement results, measurement was performed five times, and the average dynamic friction coefficient was calculated to obtain the average value thereof.

[Test method 2]

A sample was produced by filling a metal dish with a measurement sample and then hardening the measurement sample. The cosmetic composition was slid by using a static or dynamic friction measuring machine (produced by Trinity-Lab Inc., TL201Ts), at room temperature of 22°C to 25°C and relative humidity of 45% to 55%, by employing a urethane tactile contactor (contact area: 1.5 $cm^2$) under the conditions of a sliding speed of 10 mm/sec, a sliding distance of 10 mm, and a vertical load of 30 g, and the surface friction was measured. This was performed five times, each average dynamic friction coefficient of the measured values of the dynamic friction coefficient at 5 times in a sliding distance of 3 to 9 mm was calculated, and the average value thereof was obtained. The average value of the dynamic friction coefficient was calculated by the same method as Test Method 1 described above.

(Measurement of contact angle of cosmetic composition)

The double-sided tape was fixed, and the cosmetic composition was uniformly applied to the adhesive surface. 5 μL of water was dropwise added onto the applied cosmetic composition with a micropipette and left to stand. One minute after the standing, water droplets were photographed with a digital camera, and the contact angle between the water droplets and the cosmetic composition application surface was measured from the photographed images.

(Evaluation of anti-corrosion properties of cosmetic composition)

The anti-corrosion properties of the cosmetic composition were evaluated by the number of viable bacteria. Specifically, 1.0 g of the cosmetic composition and sterilized physiological saline (0.84% aqueous sodium chloride solution) were added to a dilution bottle and appropriately diluted. After this was well-suspended, 1 mL was dropwise added to a petri film for general bacterial testing and incubated at 35°C for 48 hours, and the numbers of viable bacteria immediately after manufacturing the cosmetic composition, and after standing at 25°C and humidity of 45% for three months were measured.

(Measurement of reflection intensity distribution of cosmetic composition)

The intensity distribution of the reflected light when the surface uniformly applied with about 0.5 g of the powder of the measurement target is irradiated with light having an incident angle of 45° was measured in the range of -90° to 90° by using a three-dimensional goniophotometer (produced by Murakami Color Research Laboratory Co., Ltd., GP-200).

(Measurement of specific surface area of cosmetic composition)

Nitrogen gas was used as the adsorption gas, about 1 g of the powder of the measurement sample was dried in a nitrogen atmosphere at 50°C for 240 minutes, and the measurement was performed using a fully automatic BET specific surface area meter (produced by Mountech Co.Ltd., Macsorb (registered trademark)).

(Measurement of adhered amount of cosmetic product to brush)

A sample was produced by filling a metal plate with a measurement sample and hardening the measurement sample. Sweeping 3 cm in one direction with a brush (the length of the brush part was 30 mm, the width was 20 mm, and the thickness was 10 mm) was repeated 10 times, and the mass of the measurement sample on the brush was measured using an electronic balance for analysis (produced by A&D Company, Limited, HR-150AZ).

(Sensory evaluation of cosmetic product)

A foundation was obtained by mixing 0.6 g of a cosmetic composition, 4.5 g of a mixture of mica, silica, titanium dioxide, and zinc oxide as a base powder, and 0.9 g of a colorant. Sensory evaluation was performed about the obtained foundation. The evaluation items were "ease of spreading", "ease of sticking", "skin compatibility", "smoothness" and "moisture sensation". Each of the evaluation items was evaluated by 10 evaluators, and the sensation of use was

evaluated in 5 levels with 5 being good and 1 being poor. About each item, a case where the average value was 4 or more was determined that the sensation of use was good.

(Example 1)

Corn starch (produced by Japan Corn Starch Co., Ltd., Y-3P, water content: 12.7%) was used as a starch particle, and zinc stearate (produced by Nitto Kasei Kogyo Co., Ltd.) was used as a lubricant. 1,900 g of corn starch and 100 g of zinc stearate (5 mass% with respect to the total mass of the corn starch and zinc stearate) were mixed by a dry compounding machine (produced by Nippon Coke & Engineering Co., Ltd., FM20C / ICP specification) at a rotation speed of 3,960 rpm for 10 minutes to obtain a cosmetic composition. 20°C of cooling water passed through the device, and the temperature during the mixing treatment was 30°C to 40°C.

(Example 2)

A cosmetic composition was obtained in the same manner as in Example 1, except that the amount of zinc stearate was 210 g (10 mass% with respect to the total mass of the corn starch and zinc stearate).

(Example 3)

A cosmetic composition was obtained in the same manner as in Example 1, except that the amount of zinc stearate was 335 g (15 mass% with respect to the total mass of the corn starch and zinc stearate).

(Example 4)

Corn starch (produced by Japan Corn Starch Co., Ltd., Y-3P, water content: 12.7%) was used as a starch particle, and zinc stearate (produced by Nitto Kasei Kogyo Co., Ltd.) was used as a lubricant. 3,800 g of corn starch, 200 g of zinc stearate (5 mass% with respect to the total mass of the corn starch and zinc stearate) were treated by a high shear mixer (produced by Nippon Coke & Engineering Co., Ltd., FM20C / IFM specification) at a rotation speed of 4,900 rpm for 10 minutes to obtain a cosmetic composition. The temperature during the mixing treatment was 61°C.

(Example 5)

A cosmetic composition was obtained in the same manner as in Example 4 except that the amount of zinc stearate was changed to 422 g (10 mass% with respect to the total mass of the corn starch and the zinc stearate).

(Example 6)

A cosmetic composition was obtained in the same manner as in Example 4 except that the amount of zinc stearate was changed to 671 g (15 mass% with respect to the total mass of the corn starch and the zinc stearate).

(Example 7)

A cosmetic composition was obtained in the same manner as in Example 1 except that rice starch (produced by Japan Corn Starch Co., Ltd., rice starch for foods, water content: 8.9%) was used as a starch particle, and the amount of zinc stearate was changed to 210 g (10 mass% with respect to the total mass of the corn starch and the zinc stearate).

(Example 8)

A cosmetic composition was obtained in the same manner as in Example 1 except that the amount of zinc stearate was changed to 39 g (2 mass% with respect to the total mass of corn starch and zinc stearate).

(Example 9)

A cosmetic composition was obtained in the same manner as in Example 4 except that a hot water (75°C) jacket was mounted to the high shear mixer such that the temperature during the mixing treatment was 80°C or higher.

(Example 10)

Corn starch (produced by Japan Corn Starch Co., Ltd., Y-3P, water content: 12.7%) was used as a starch particle, and zinc stearate (produced by Nitto Kasei Kogyo Co., Ltd.) was used as a lubricant. 29.1 g of corn starch and 0.9 g of zinc stearate (3 mass% with respect to the total mass of corn starch and zinc stearate) were mixed with a mortar for 10 minutes to obtain a cosmetic composition. The zinc stearate adhesion was observed on the surface of the corn starch, but particles of zinc stearate alone remained in the cosmetic composition, and not all zinc stearate covered the corn starch surface.

(Example 11)

A cosmetic composition was obtained in the same manner as in Example 10 except that the amount of the corn starch was changed to 28.5 g, and the amount of zinc stearate was changed to 1.5 g (5 mass% with respect to the total mass of the corn starch and zinc stearate).

(Example 12)

A cosmetic composition was obtained in the same manner as in Example 1 except that the amount of zinc stearate was changed to 59 g (3 mass% with respect to the total mass of the corn starch and zinc stearate).

(Comparative Example 1)

An untreated corn starch (produced by Japan Corn Starch Co., Ltd., Y-3P, water content: 12.7%) was used as the composition of Comparative Example 1.

(Comparative Example 2)

A composition of Comparative Example 2 was obtained by not adding zinc stearate to 2,000 g of corn starch (produced by Japan Corn Starch Co., Ltd., Y-3P, water content: 12.7%) and performing a treatment by a dry compounding machine (produced by Nippon Coke & Engineering Co., Ltd., FM20C / ICP specification) under the same condition as in Example 1.

The results of flowability evaluation on the cosmetic compositions of Examples 1 to 6 and 9 and Comparative Example 1 based on the Carr's index table are shown in Tables 1-1 and 1-2.

[Table 1-1]

| | Comparative Example 1 | | Example 1 | | Example 2 | | Example 3 | |
|---|---|---|---|---|---|---|---|---|
| | Water content: 12.7% | | Water content: 11.6% | | Water content: 10.2% | | Water content: 9.7% | |
| | Result | Index | Result | Index | Result | Index | Result | Index |
| Degree of compression (%) | 49.1 | 0 | 32.9 | 7.0 | 32.0 | 9.5 | 31.5 | 9.5 |
| Angle of repose (°) | 55.8 | 9.5 | 40.2 | 17.5 | 39.0 | 18.0 | 38.5 | 18.0 |
| Angle of spatula (°) | 66.3 | 12.0 | 51.5 | 16.0 | 51.4 | 16.0 | 48.5 | 16.0 |
| Degree of uniformity | 2.2 | 23.0 | 2.2 | 23.0 | 2.2 | 23.0 | 2.2 | 23.0 |
| Total index (Flowability index) | 44.5 | | 63.5 | | 66.5 | | 66.5 | |
| Evaluation of flowability | Low | | Normal | | Normal | | Normal | |

[Table 1-2]

| | Example 4 | | Example 5 | | Example 6 | | Example 9 | |
|---|---|---|---|---|---|---|---|---|
| | Water content: 11.6% | | Water content: 11.0% | | Water content: 10.6% | | Water content: 9.7% | |
| | Result | Index | Result | Index | Result | Index | Result | Index |
| Degree of compression (%) | 30.3 | 12.0 | 31.7 | 9.5 | 33.4 | 7.0 | 31.7 | 9.5 |
| Angle of repose (°) | 32.7 | 21.0 | 38.0 | 18.0 | 39.3 | 18.0 | 38.6 | 18.0 |
| Angle of spatula (°) | 41.2 | 18.0 | 42.1 | 18.0 | 37.3 | 21.0 | 45.0 | 17.5 |
| Degree of uniformity | 2.2 | 23.0 | 2.2 | 23.0 | 2.2 | 23.0 | 2.2 | 23.0 |
| Total index (Flowability index) | 74.0 | | 68.5 | | 69.0 | | 68.0 | |
| Evaluation of flowability | High | | Normal | | Normal | | Normal | |

(Flowability evaluation on cosmetic composition based on the Carr's index table)

The flowability index of the cosmetic compositions of Examples 1 to 6 and 9 was 1.43 to 1.66 times of that of the cosmetic composition of Comparative Example 1. The evaluation of flowability was "low" in the cosmetic composition of Comparative Example 1 but was "normal" in the cosmetic compositions of Examples 1 to 3, 5, 6, and 9, and was improved to "high" in Example 4. From the above, it was confirmed that the flowability was improved by covering the surface of the starch particle with the lubricant as compared with the case of the starch particle alone.

(Effect of temperature in mixing treatment)

In addition, the influence of temperature in the mixing treatment was examined. FIGS. 2 to 4 are SEM images of the cosmetic compositions of Examples 1, 4, and 9, respectively. In the cosmetic composition of Example 4 in which the maximum temperature in the mixing treatment was 61°C, it was confirmed that the particle shape of the lubricant remained on the surface. Meanwhile, in the cosmetic composition of Example 9 in which the maximum temperature in the mixing treatment was 90°C, the particle shape of the lubricant was not confirmed, and it was confirmed that the surface state was the same as that of the cosmetic composition of Example 1.

The lubricant in the cosmetic compositions of Examples 1, 4 and 9 was zinc stearate and with a melting point of 120°C. In Example 9, it was confirmed that by setting the temperature in the mixing treatment to be 80°C to 90°C, the lubricant was softened, and the lubricant was sufficiently ground and covered the surface of the starch particle. Therefore, by performing the mixing treatment at a high temperature that does not reach the melting point of the lubricant, the lubricant is softened, even if a shearing force as high as that of the dry compounding machine was not applied, the lubricant was sufficiently ground and covered the starch particle surface.

(Particle size distribution of cosmetic composition)

FIG. 5 shows the particle size distribution of the cosmetic composition of Example 12 (indicated by circles) and the untreated corn starch of Comparative Example 1 (indicated by triangles). In the untreated corn starch of Comparative Example 1, since the secondary particles which are aggregates of primary particles were included, particles with a particle diameter of about 20 to 40 μm were observed. In Comparative Example 1, the particle diameter $D_{10}$ was 11.69 μm, the particle diameter $D_{50}$ was 16.95 μm, and the particle diameter $D_{90}$ was 25.81 μm. The difference between the particle diameter $D_{10}$ and the particle diameter $D_{90}$ of Comparative Example 1 was 14.12 μm. The particle diameter corresponding to the mode of the particle size distribution of Comparative Example 1 was 14.99 μm.

Meanwhile, in the cosmetic composition of Example 12, particles with a particle diameter of about 20 to 40 μm were not confirmed, and a particle size distribution having a sharp peak compared to Comparative Example 1 was obtained. The particle diameter $D_{10}$ of Example 12 was 11.47 μm, the particle diameter $D_{50}$ was 15.16 μm, and the particle diameter $D_{90}$ was 19.76 μm. The difference between the particle diameter $D_{10}$ and the particle diameter $D_{90}$ of Example 12 was 8.29 μm. The particle diameter corresponding to the mode of the particle size distribution of Example 12 was 14.99 μm.

The reason why the particle size distribution having a sharper peak than in Comparative Example 1 was obtained is that secondary particles of corn starch were pulverized in the step of covering corn starch, which is a starch particle,

with a lubricant.

(Average friction coefficient of cosmetic composition)

The results of measuring the average friction coefficient of the cosmetic compositions of Examples 1, 2, 4, and 5 were 0.48, 0.42, 0.48, and 0.53, respectively. The results of measuring the average friction coefficient of the cosmetic composition of Comparative Example 1 and spherical nylon (produced by Toray Industries, Inc., SP-500) were 0.62 and 0.48.

In the cosmetic composition of Example 5, the average friction coefficient was increased compared to the spherical nylon, but decreased by about 15% as compared with the cosmetic composition of Comparative Example 1. In the cosmetic compositions of Examples 1, 2, and 4, the average friction coefficient was decreased by 23% to 32% compared to the cosmetic composition of Comparative Example 1 and was below the average friction coefficient of spherical nylon.

From the foregoing, it was confirmed that, by covering the surface of the starch particles with the lubricant, the average friction coefficient of the cosmetic composition was smaller than that of the starch particles alone, and it was confirmed that the average friction coefficient of the cosmetic composition was similar to or less than that of the spherical nylon.

The reason why the average friction coefficient in the cosmetic composition of Example 5 was larger than that of the other examples was because not all the lubricant adhered to the surface of the starch particles, and only the lubricant was present, so the frictional force was increased by the particles of the lubricant alone.

(Average dynamic friction coefficient of cosmetic composition)

Average dynamic friction coefficients of the cosmetic compositions of Example 10 and Example 12, Comparative Example 1, Mica (produced by Yamaguchi Mica Co., Ltd.), zinc stearate (produced by Nitto Chemical Industry Co., Ltd.), spherical nylon (manufactured by Toray Industries, Inc., SP-500), and polymethyl methacrylate (PMMA, produced by Toagosei Co., Ltd.) are shown in Table 2. These average dynamic friction coefficients were measured by Test Method 1.

[Table 2]

| Name of sample | Average dynamic friction coefficient |
|---|---|
| Example 10 | 0.428 |
| Example 12 | 0.406 |
| Comparative Example 1 | 0.537 |
| Mica | 0.588 |
| Zinc stearate | 0.476 |
| Spherical nylon | 0.421 |
| PMMA | 0.31 |

Mica is a plate-like particle that is generally used as a main component of a solid cosmetic product. The cosmetic compositions of Examples 10 and 12 had small average dynamic friction coefficients of about 27% and 31%, respectively, and slipperiness was high, compared with mica. The average dynamic friction coefficients of the cosmetic compositions of Examples 10 and 12 were about 20% and 24% smaller, respectively, compared with Comparative Example 1. The average dynamic friction coefficients of the cosmetic compositions of Examples 10 and 12 were about 10% and 15% smaller, respectively, compared to zinc stearate.

Nylon and PMMA are true spherical plastic particles blended into the cosmetic product of the solid preparation for the purpose of improving slipperiness. The cosmetic compositions of Examples 10 and 12 had average dynamic friction coefficients similar to or lower than that of nylon.

When Examples 10 and 12 were compared with each other, it was confirmed that the average dynamic friction coefficient of Example 12 was about 5% smaller. In a case where the lubricant coated the corn starch surface to be composited, slipperiness was higher than in a case where the lubricant was simply adhered to the corn starch surface.

(Contact angle of cosmetic composition)

The measurement results of the contact angles of the cosmetic compositions of Examples 1, 4, and 8 were

117°, 125°, and 74°, respectively. In Comparative Example 1, water droplets were absorbed simultaneously with contact with corn starch. As a result, it was confirmed that if the starch particles were coated with the lubricant, the contact angle of the water droplets was large and water repellency was exhibited. In addition, with a comparison between Examples 1 and 8, it was confirmed that, as the lubricant ratio was larger, the contact angle was larger.

(Anti-corrosion properties of cosmetic composition)

Table 3-1 shows the results of the evaluation of anti-corrosion properties of the cosmetic composition of Example 7 and rice starch (for food).

[Table 3-1]

| | Number of viable bacteria (cfu/g) | |
|---|---|---|
| | Immediately after production | After 3 months |
| Example 7 | 1413 | 1161 |
| Rice starch | 1834 | 1877 |

Comparing the cosmetic composition of Example 7 of which the starch particles were rice starch with rice starch, the number of viable bacteria immediately after production was 23% less in the cosmetic composition of Example 7, the number of viable bacteria after 3 months later from the production was 38% fewer in the cosmetic composition of Example 7. It is considered that water repellency and hydrophobicity were improved by covering the surface of the starch particles of the cosmetic composition of Example 7 with the lubricant, so bacterial growth was suppressed. From the foregoing, it was confirmed that the anti-corrosion properties were imparted by covering the starch particle surface of the cosmetic composition with the lubricant.

(Measurement of reflection intensity of cosmetic composition)

The reflection intensity distribution of the cosmetic composition of Example 12 was measured. In addition to the cosmetic composition of Example 12, the reflection intensity distribution was measured for mica, spherical nylon, and PMMA. The results thereof are provided in FIG. 6. The intensity of the reflected light is expressed as a relative value when the maximum reflection intensity in the cosmetic composition of Example 12 was 85%.

In the cosmetic product such as foundations, for the purpose of causing the wrinkles and irregularities of the skin to be less noticeable, it is preferable that the particles included in the foundations or the like have irregular reflectivity, a so-called soft focus effect.

Since mica is a plate-like particle, incident rays are almost regularly reflected. PMMA has irregular reflectivity and has a soft focus effect, but has weak reflection intensity near a reflection angle of 0°.

The cosmetic composition of Example 12 has irregular reflectivity, and has stronger reflection intensity near a reflection angle of 0° than PMMA, so it can be said that the cosmetic composition of Example 12 has a higher soft focus effect than PMMA.

(Specific surface area of cosmetic composition)

The specific surface areas of Examples 1, 11, and 12 and Comparative Examples 1 and 2 were measured. The results are shown in Table 3-2.

[Table 3-2]

| Name of sample | Specific surface area ($m^2/g$) |
|---|---|
| Example 1 | 0.814 |
| Example 11 | 0.996 |
| Example 12 | 0.635 |
| Comparative Example 1 | 0.585 |
| Comparative Example 2 | 0.551 |

In Examples 1 and 12 in which zinc stearate covered the corn starch surface, as the proportion of zinc stearate increases (increase from 3 mass% to 5 mass%), the specific surface area tends to increase (increase from 0.635 $m^2/g$ to 0.814 $m^2/g$).

Comparing Example 1 with Example 11, the proportions of zinc stearate were the same and were 5%, but the specific surface area of Example 11 was 0.996 $m^2/g$. As a cause of the difference in specific surface areas between Examples 1 and 11, Example 1 is a state in which zinc stearate covered the corn starch surface, and zinc stearate is composited with corn starch, but in Example 11, it is considered that the particles of zinc stearate alone remained and not all the zinc stearate covered the corn starch surface.

The specific surface areas of Comparative Examples 1 and 2 were almost the same but were smaller than those of Examples 1 and 12. In Comparative Example 2, it was thought that the secondary particles of corn starch were pulverized by the dry compounding machine to increase the specific surface area, but it is considered that, in the absence of a lubricant such as zinc stearate, the pulverized particles were aggregated again to cause the specific surface area to be smaller than those of Examples 1 and 12.

(Coefficient of average dynamic friction of cosmetic product)

(1) Eye shadow

The average dynamic friction coefficients of eye shadows when the cosmetic compositions of Examples 12 and 10 were blended as a feel modifier of the eye shadows was measured in accordance with Test Method 1. In addition to the cosmetic compositions of Examples 12 and 10, the average dynamic friction coefficient of eye shadow was measured by using corn starch, zinc stearate, spherical nylon, and PMMA of Comparative Example 1 as the feel modifier. Table 4-1 shows blending ratios of each component included in the eye shadow, and Table 4-2 shows average dynamic friction coefficients of eye shadows not including the feel modifier and eye shadows including each feel modifier.

[Table 4-1]

| Raw material | Without feel modifier (mass%) | With feel modifier (mass%) |
|---|---|---|
| Mica | 73.8 | 68.8 |
| Kaolin | 9.8 | 9.8 |
| Titanium dioxide | 4.3 | 4.3 |
| Jojoba oil | 6.5 | 6.5 |
| Colorant | 5.6 | 5.6 |
| Feel modifier | 0 | 5.0 |
| (Total) | 100.0 | 100.0 |

[Table 4-2]

| Feel modifier | Average friction coefficient |
|---|---|
| Example 10 | 0.613 |
| Example 12 | 0.583 |
| Without feel modifier | 0.639 |
| Comparative Example 1 | 0.618 |
| Zinc stearate | 0.655 |
| Spherical nylon | 0.581 |
| PMMA | 0.578 |

The eye shadows including the cosmetic compositions of Examples 12 and 10 had average dynamic friction coefficients lower than the eye shadows not including a feel modifier by 9% and 4%, respectively. Particularly, it was found that, in the eye shadow including the cosmetic composition of Example 12, the average dynamic friction coefficient decreased to the same degree as spherical nylon and PMMA, which have been used as conventional feel modifiers.

Moreover, it was found that the single substance of zinc stearate cannot obtain the slipperiness improvement effect as a feel modifier.

(2) Blusher

The average dynamic friction coefficient of a blusher when the cosmetic composition of Example 8 was blended as a feel modifier of the blusher was measured in accordance with Test Method 2. The average dynamic friction coefficient of a blusher using spherical nylon as a feel modifier was also measured. The blending ratio of each component included in the blusher was 52.5 mass% or 47.5 mass% of mica as the main component, 13 mass% of the colorant, 5 mass% or 10 mass% of the feel modifier, and 29.5 mass% as other components with respect to the total mass of the blusher. The average dynamic friction coefficient of each blusher is shown in Table 4-3.

[Table 4-3]

| Feel modifier | Average friction coefficient |
|---|---|
| Example 8 (5 mass%) | 0.512 |
| Example 8 (10 mass%) | 0.500 |
| Spherical nylon (5 mass%) | 0.508 |
| Spherical nylon (10 mass%) | 0.533 |

It was confirmed that the blusher obtained by blending the cosmetic composition of Example 8 had an average dynamic friction coefficient to the same degree as that of the blusher obtained by blending spherical nylon. Further, it was confirmed that, when the blending ratio of spherical nylon was 10 mass%, the average dynamic friction coefficient was increased. On the other hand, when the blending ratio of the cosmetic composition of Example 8 was 10 mass%, the average dynamic friction coefficient was further reduced and it was suggested that ease of spreading of the blusher was improved.

(Measurement of adhered amount of cosmetic product to brush)

By using the blusher used in "Dynamic friction coefficient of cosmetic product" (2), the adhered amount of cosmetic product to the brush was measured. The results are shown in Table 4-4.

[Table 4-4]

| Feel modifier | Adhered amount of cosmetic product (mg) |
|---|---|
| Example 8 (5 mass%) | 2.72 |
| Example 8 (10 mass%) | 4.08 |
| Spherical nylon (5 mass%) | 1.56 |
| Spherical nylon (10 mass%) | 1.66 |

Compared with the cosmetic product including spherical nylon, in the cosmetic product including the cosmetic composition of Example 8, the adhered amount of the brush was increased. In addition, the adhered amount of the cosmetic product to the brush was increased by increasing the blending ratio of the cosmetic composition of Example 8 in the cosmetic product. Meanwhile, even if the proportion of spherical nylon was increased, the adhered amount of the cosmetic product to the brush hardly increased.

In the cosmetic product that is obtained by hardening the powder with a press or the like, such as a blusher used as a sample, the resistance to cracking is important. By blending the cosmetic composition of Example 8 into such a cosmetic product, it is possible to improve the adhered amount to the brush while the resistance to cracking was maintained.

(Sensory evaluation of cosmetic product)

Table 4-5 shows sensory evaluation results of cosmetic products using Example 1, Comparative Example 1, and spherical nylon (SP-500, manufactured by Toray Industries, Inc.) as the cosmetic compositions.

[Table 4-5]

| | Spherical nylon | Comparative Example 1 | Example 1 |
|---|---|---|---|
| Ease of spreading | 4.5 | 2.8 | 4.3 |
| Ease of sticking | 4.2 | 3.0 | 4.2 |
| Skin compatibility | 4.0 | 2.5 | 4.5 |
| Smoothness | 3.2 | 2.7 | 4.6 |
| Moisture sensation | 3.0 | 3.1 | 4.8 |
| Average value of total items | 3.8 | 2.8 | 4.5 |

In the cosmetic product using spherical nylon, evaluations of "ease of spreading" and "ease of sticking" were good, but evaluations of "moisture sensation" were inferior. In the cosmetic product using Comparative Example 1, overall evaluations were low, as a result. In the cosmetic product using Example 1, compared with the cosmetic product using spherical nylon, the evaluation was high in the items "f skin compatibility", "smoothness", and "moisture sensation", and all items were evaluated to be as high as 4 or more.

Industrial Applicability

According to the present invention, it is possible to provide a naturally derived material cosmetic composition which can be efficiently produced, has good spreadability on the skin, and is excellent in anti-corrosion properties, a cosmetic including the cosmetic composition, and a method for producing the cosmetic composition.

Reference Signs List

1... cosmetic composition, 11... starch particle, 12... lubricant layer

## Claims

**1.** A cosmetic composition, comprising:

a starch particle; and
a lubricant that covers at least a portion of a surface of the starch particle and containing a fatty acid,
wherein a mass of the lubricant with respect to the total mass of the starch particle and the lubricant is 0.3 mass% to 20 mass%.

**2.** The cosmetic composition according to claim 1,
wherein the lubricant is at least one substance selected from the group consisting of metal soaps and waxes derived from vegetable oils and fats.

**3.** The cosmetic composition according to claim 1 or 2,
wherein an average friction coefficient of the cosmetic composition is 0.30 to 0.55.

**4.** The cosmetic composition according to any one of claims 1 to 3,
wherein an average particle diameter of the cosmetic composition is at least 2 $\mu$m but not more than 80 $\mu$m.

**5.** The cosmetic composition according to any one of claims 1 to 4,
wherein a flowability index of the cosmetic composition based on the Carr’s index table is at least 60.

**6.** The cosmetic composition according to any one of claims 1 to 5,
wherein the lubricant covers at least 90% of the surface of the starch particle.

**7.** The cosmetic composition according to any one of claims 1 to 6,
wherein the starch particle is corn starch, and the lubricant is zinc stearate.

**8.** The cosmetic composition according to any one of claims 1 to 7, wherein a specific surface area of the cosmetic composition is at least 0.60 $m^2/g$ but not more than 0.85 $m^2/g$.

**9.** The cosmetic composition according to any one of claims 1 to 8, wherein an average friction coefficient of the cosmetic composition is 0.30 to 0.55.

**10.** The cosmetic composition according to any one of claims 1 to 9, wherein an average particle diameter of the cosmetic composition is at least 10 μm but not more than 20 μm.

**11.** The cosmetic composition according to any one of claims 1 to 10, wherein the starch particle and the lubricant are composited.

**12.** A cosmetic product comprising: the cosmetic composition according to any one of claims 1 to 11.

**13.** A method for producing a cosmetic composition, comprising: refining particles of a lubricant containing a fatty acid and covering at least a portion of a surface of a starch particle with the lubricant by dry-mixing the starch particle and the lubricant.

**14.** A method for producing a cosmetic composition according to claim 13, wherein the starch particle and the lubricant are dry-mixed such that a mass of the lubricant is 0.3 mass% to 20 mass% with respect to the total mass of the starch particle and the lubricant.

**15.** The method for producing a cosmetic composition according to claim 13 or 14, wherein the starch particle and the lubricant are dry-mixed by using a dry compounding machine or a high shear mixer.

**16.** The method for producing a cosmetic composition according to any one of claims 13 to 15, wherein an average particle diameter of the lubricant before the dry-mixing is 1.0 μm or more.

**17.** The method for producing a cosmetic composition according to any one of claims 13 to 16, wherein the dry-mixing is performed at 30°C or more.

**18.** The method for producing a cosmetic composition according to any one of claims 13 to 17, wherein the starch particle is corn starch, and the lubricant is zinc stearate.

**19.** The method for producing a cosmetic composition according to any one of claims 13 to 18, wherein the dry-mixing is performed at 30°C to 90°C.

*FIG. 1*

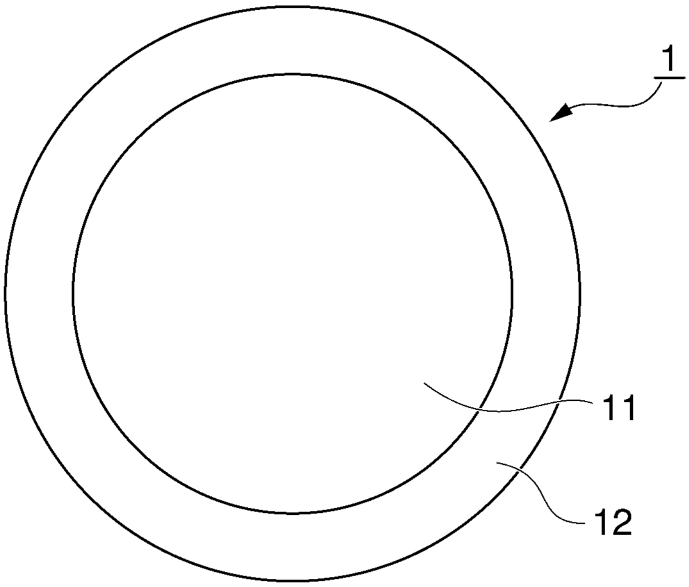


*FIG. 2*

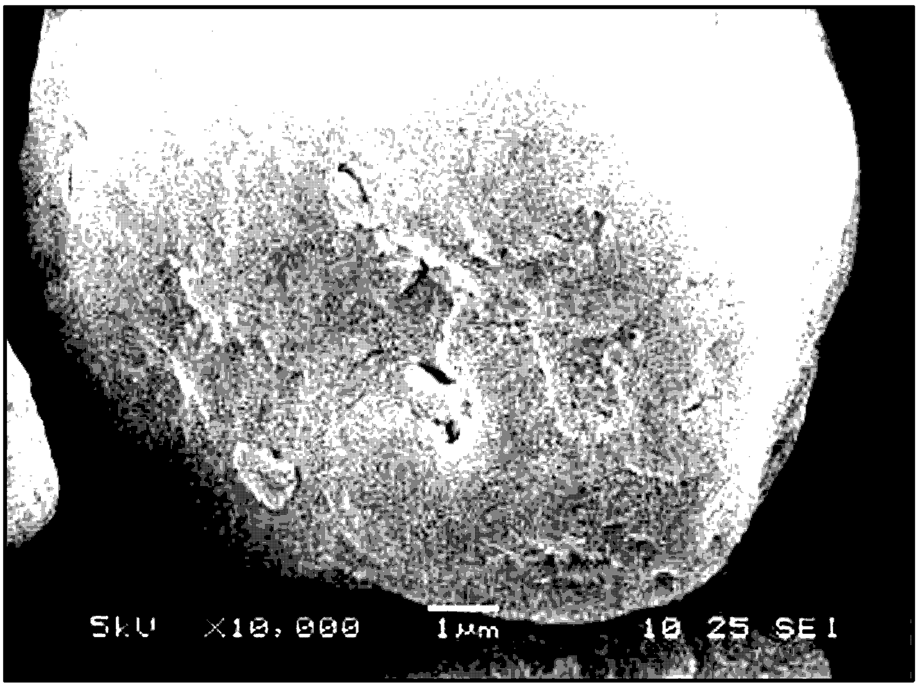

FIG. 3

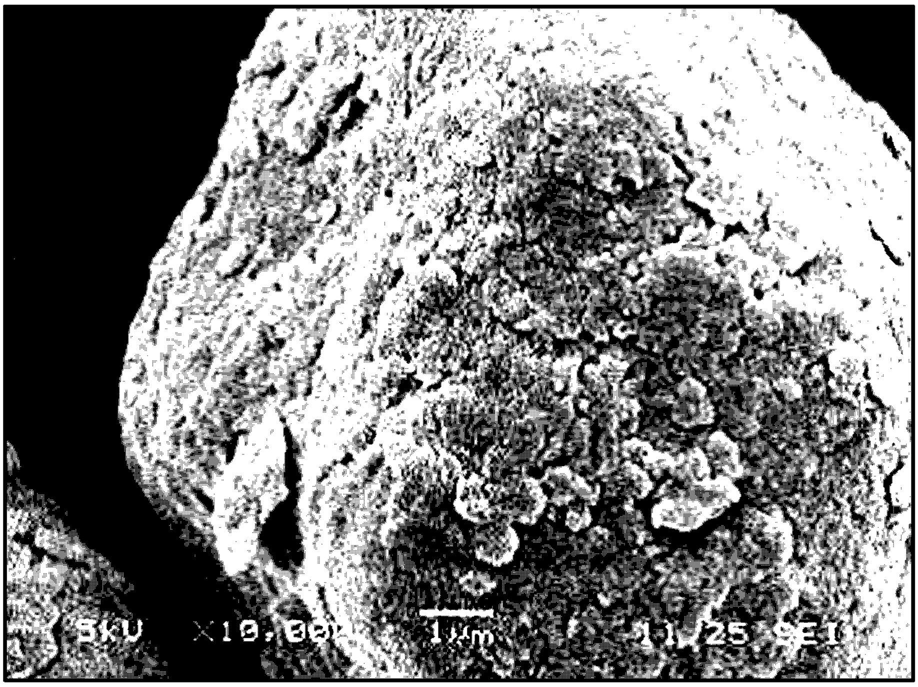


FIG. 4

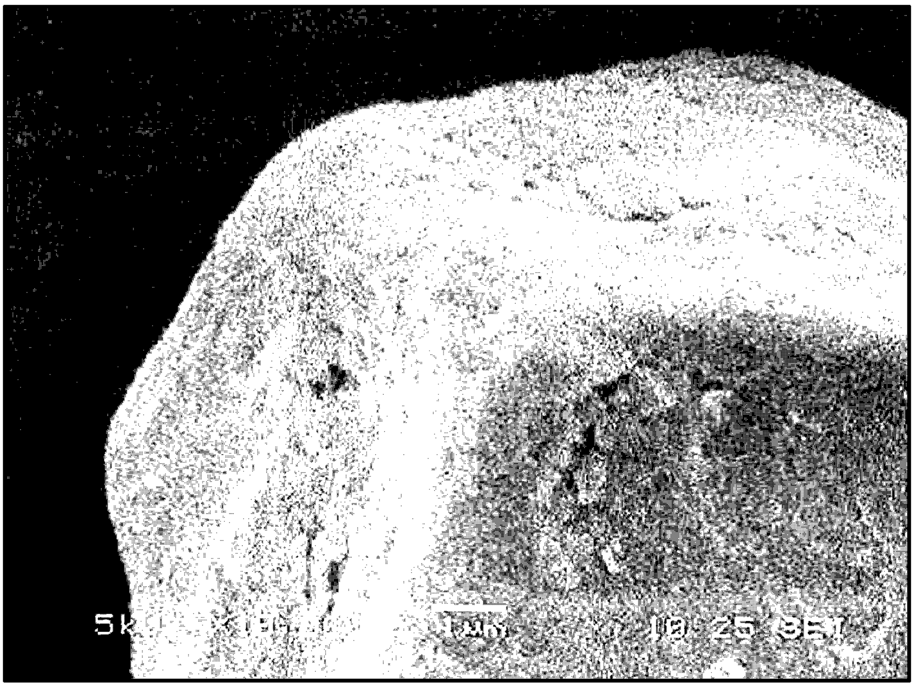

FIG. 5

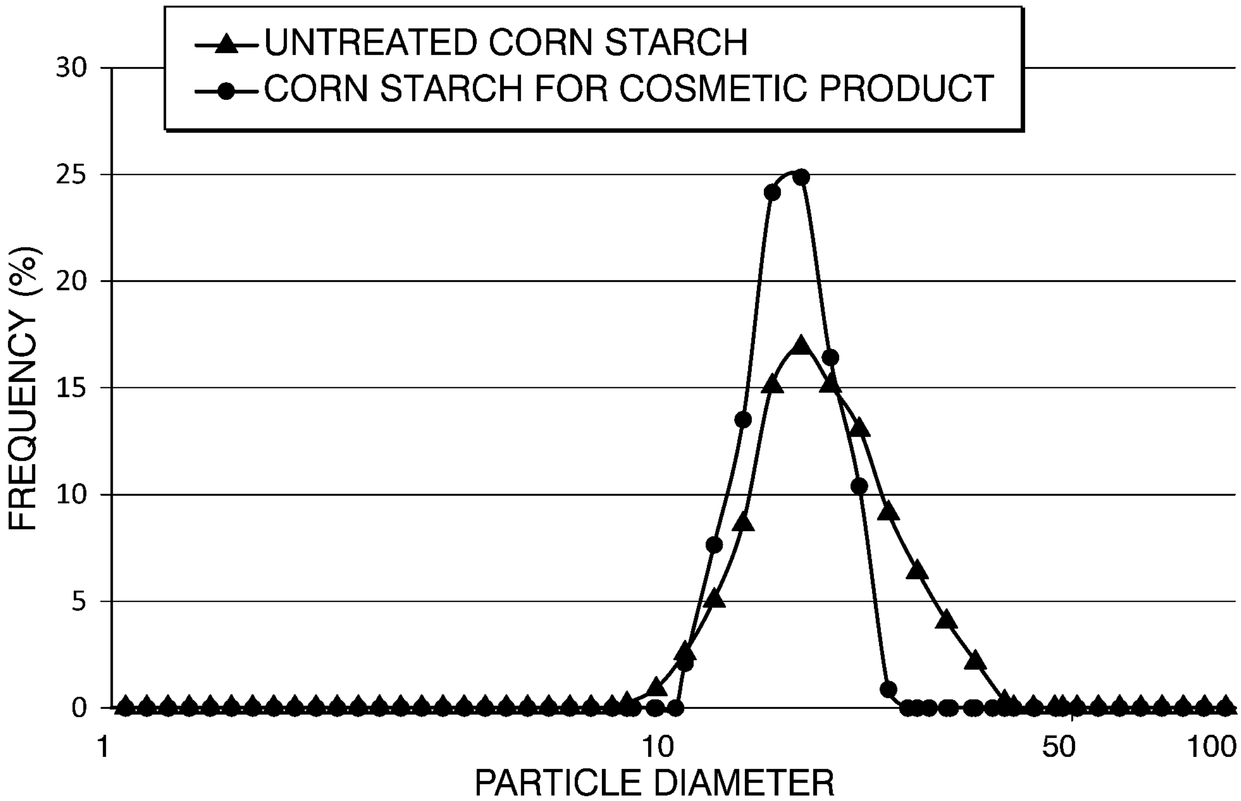
UNTREATED CORN STARCH
CORN STARCH FOR COSMETIC PRODUCT
FREQUENCY (%)
30
25
20
15
10
5
0
1
10
50
100
PARTICLE DIAMETER

FIG. 6

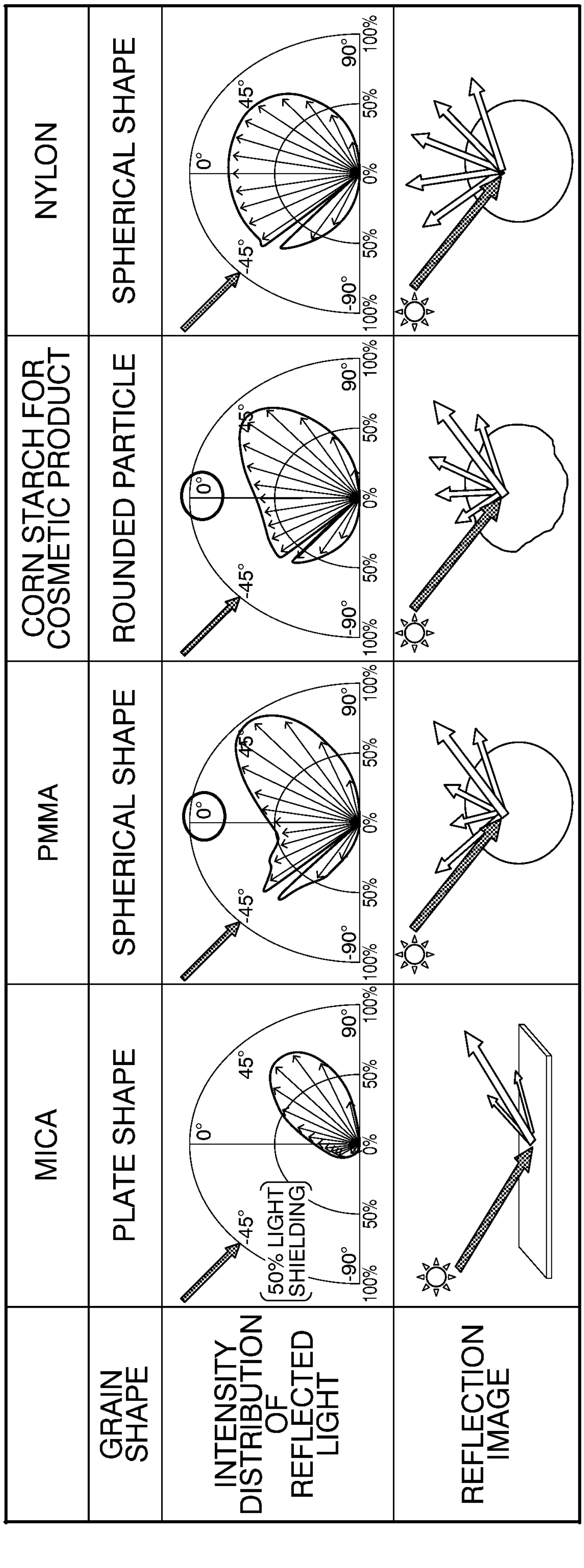
GRAIN SHAPE
INTENSITY DISTRIBUTION OF REFLECTED LIGHT
REFLECTION IMAGE
MICA
PLATE SHAPE
(50% LIGHT SHIELDING)
PMMA
SPHERICAL SHAPE
CORN STARCH FOR COSMETIC PRODUCT
ROUNDED PARTICLE
NYLON
SPHERICAL SHAPE
-90°
-45°
0°
45°
90°
100%
50%
0%
50%
100%

## INTERNATIONAL SEARCH REPORT

International application No.
PCT/JP2018/047831

A. CLASSIFICATION OF SUBJECT MATTER
Int.Cl. A61K8/73(2006.01)i, A61Q1/02(2006.01)i
According to International Patent Classification (IPC) or to both national classification and IPC

B. FIELDS SEARCHED
Minimum documentation searched (classification system followed by classification symbols)
Int.Cl. A61K8/73, A61Q1/02

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | KR 10-2011-0099370 A (LEE Myong-Il) 08 September 2011, claims, paragraphs [0001]-[0005], [0013]-[0015], examples (Family: none) | 1-9 |
| X | KR 10-2013-0083306 A (KOLMAR KOREA CO., LTD.) 22 July 2013, claims, paragraphs [0017]-[0020], [0027], [0030], [0036]-[0041], examples (Family: none) | 1-2, 7, 11-19 |
| X | JP 2014-240479 A (CHIBA FLOUR MILLING CO., LTD.) 25 December 2014, claims, paragraphs [0001]-[0006], [0017]-[0034], examples & CN 104151593 A | 1-12 |
| X | JP 2005-263709 A (KAO CORP.) 29 September 2005, example 6 (Family: none) | 13 |
| X | JP 63-215616 A (ASADA MILLING CO., LTD.) 08 September 1988, claims, page 2, upper left column to lower left column, examples (Family: none) | 1-6, 8-9, 11-12 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 25 March 2019 (05.03.2019) | 02 April 2019 (02.04.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2018/047831

C (Continuation). DOCUMENTS CONSIDERED TO BE RELEVANT

| Category* | Citation of document, with indication, where appropriate, of the relevant passages | Relevant to claim No. |
|---|---|---|
| A | JP 2014-533252 A (GIVAUDAN SA) 11 December 2014, entire text & US 2014/0294736 A1 & WO 2013/068581 A2 & EP 2775990 A2 | 1-19 |
| P, X | JP 2018-76277 A (DAITO KASEI KOGYO CO., LTD.) 17 May 2018, claims, paragraphs [0001]-[0007], [0009]-[0016], production examples 1-2 (Family: none) | 1-12 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2017249854 A
- JP 2003146829 A
- JP 2017052706 A


### Non-patent literature cited in the description


- **CARR, R. L.** Evaluating flow properties of solids. *Chem. Eng.,* 1965, vol. 72, 163-168